# Europäisches Patentamt
# European Patent Office
# Office européen des brevets

(19)

(11) Veröffentlichungsnummer: **0 257 202**
**B1**

(12) **EUROPÄISCHE PATENTSCHRIFT**

(45) Veröffentlichungstag der Patentschrift:
**06.12.89**

(21) Anmeldenummer: **87107243.5**

(22) Anmeldetag: **19.05.87**

(51) Int. Cl.⁴: **C07C 51/235**, C07C 55/21,
C07C 45/50, B01J 23/34

(54) Verfahren zur Herstellung von 1,12-Dodecandisäure I.

(30) Priorität: **23.08.86 DE 3628662**

(43) Veröffentlichungstag der Anmeldung:
**02.03.88 Patentblatt 88/9**

(45) Bekanntmachung des Hinweises auf die Patenterteilung:
**06.12.89 Patentblatt 89/49**

(84) Benannte Vertragsstaaten:
**AT BE DE FR GB NL SE**

(56) Entgegenhaltungen:
**EP-A- 0 026 998**
**DE-A- 2 658 043**
**GB-A- 2 034 310**

**CHEMICAL ABSTRACTS, Band 107, Nr. 11, 14.
September 1987, Seite 654,
Zusammenfassungsnr. 96332m, Columbus, Ohio, US; Y.
TOKITO "Preparation of alpha,omega-dialdehydes", &
JP - A - 62 30734
PATENT ABSTRACTS OF JAPAN, Band 11,
Nr. 85 (C-410)[2532], 14. März 1987; & JP - A - 61 238 751**

(73) Patentinhaber: **Degussa Aktiengesellschaft,
Weissfrauenstrasse 9, D-6000 Frankfurt am Main 1(DE)**

(72) Erfinder: **Andrade, Juan, Dr., 545 Barnett Place,
Ridgewood N.J. 07450(US)**
Erfinder: **Köhler, Klaus, Kettelerstrasse 37,
D-6452 Hainburg(DE)**
Erfinder: **Prescher, Günter, Dr., Liesingstrasse 2,
D-6450 Hanau 9(DE)**

**Beschreibung**

Die Erfindung betrifft ein Verfahren zur Herstellung von 1,12-Dodecandisäure

Es ist bekannt, diese Säure durch Oxidation und Verseifung des bei der Hydroformylierung von Methylundecylenat entstehenden Methyl-11-aldehydo-undecanoats zu gewinnen (H. Adkins, G. Krsek, JACS, Vol. 70, Januar 1948, S. 383 - 386).

Ebenfalls nur an einer Doppelbindung hydroformylierte Produkte erhält man, wenn man 1,9-Decadien in Gegenwart einer Rhodiumverbindung als Katalysator mit Kohlenmonoxid und Wasserstoff umsetzt.

Es entstehen Undecenale (DE-OS 2 724 484), die nach Oxidation und Veresterung als Ausgangsverbindung gemäß den Verfahren von Adkins und Krsek eingesetzt werden können.

Es wurde nun ein Verfahren zur Herstellung von 1,12-Dodecandisäure durch Hydroformylierung eines Olefins in Gegenwart eines Rhodiumkatalysators und Oxidation des dabei gebildeten Aldehyds mit Sauerstoff gefunden, das dadurch gekennzeichnet ist, daß man 1,9-Decadien in Gegenwart von Hydridotris-triphenylphosphin-rhodium-carbonyl kombiniert mit Triphenylphosphin und/oder Triphenylphosphit hydroformyliert und das dabei gebildete 1,12-Dodecandial in Gegenwart von anorganischen oder organischen Co-(II)-salzen mit Sauerstoff oxidiert.

Das 1,9-Decadien wird mit einem Gasgemisch aus Wasserstoff und Kohlenmonoxid in Gegenwart eines Katalysators hydroformyliert. Die Umsetzung erfolgt zweckmäßigerweise bei Temperaturen von 70 bis 140 °C, vorzugsweise bei Temperaturen von 80 bis 120 °C. Der Druck kann weitgehend beliebig gewählt werden, jedoch ist es zweckmäßig, im Druckbereich von 1 bis 12 bar zu arbeiten. Bevorzugt werden Drücke von 2 bis 8 bar.

Zweckmäßig ist es, die zur Umsetzung mindestens notwendigen stöchiometrischen Mengen, vorzugsweise jeweils die 1,05 bis 2,5-fache Mengen, Wasserstoff und Kohlenmonoxid zu verwenden, wobei das Molverhältnis Wasserstoff zu Kohlenmonoxid weitgehend beliebig gewählt werden kann, vorzugsweise jedoch zwischen 0,5 zu 1,0 bis 1,0 zu 0,5 liegt.

Bei der Hydroformylierung dient als Katalysator Hydridotris-triphenylphosphin-rhodium-carbonyl kombiniert mit Triphenylphosphin und beziehungsweise oder Triphenylphosphit. Derartige Katalysatoren sind in der DE-AS 1793069 beschrieben. Vorzugsweise werden bei der Durchführung des erfindungsgemäen Verfahrens je Gewichtsteil des 1,9-Decadiens 0,002 bis 0,01 Gewichtsteile des Hydridotris-triphenylphosphin-rhodium-carbonyls und insgesamt 0,02 bis 0,3 Gewichtsteile des Triphenylphosphins und/oder Triphenylphosphits angewenendet.

Das bei der Hydroformylierung entstehende 1,12-Dodecandial wird in einer bevorzugten Variante des erfindungsgemäßen Verfahrens aus dem Reaktionsgemisch destillativ abgetrennt und anschließend bei 10 bis 100 °C, bevorzugt 40 bis 90 °C, in Gegenwart eines organischen oder anorganischen Co(II)-salzes mit einem sauerstoffhaltigen Gas oder reinem Sauerstoff oxidiert. Bevorzugt werden je Gewichtsteil des 1,12-Dodecandials 0,005 bis 0,09 Gewichtsteile des Co(II)-salzes, insbesondere Co(II)-acetat, verwendet.

In einer besonderen Ausführungsform setzt man der zu oxidierenden Verbindung eine Percarbonsäure in einer Menge von 0,002 bis 0,2 Gewichtsteile, bezogen auf die Verbindung, zu.

Bevorzugt eingesetzt werden Peressigsäure und Perpropionsäure.

Die entstehende 1,12-Dodecandi säure kristallisiert nach Beendigung der Umsetzung und Abkühlung aus dem Oxidationsgemisch aus und wird mit bekannten Maßnahmen abgetrennt.

Die Oxidation wird in einer besonders geeigneten Variante direkt in der bei der Hydroformylierung anfallenden Lösung durchgeführt, wobei die sonstigen Maßnahmen, wie oben beschrieben, bestehen bleiben.

Eine bevorzugte Ausführungsform beinhaltet die Verwendung eines inerten organischen Lösungsmittels, bevorzugt eines aromatischen wie z. B. Benzol oder Toluol, in dem bei der Hydroformylierung bzw. Oxidation vorgelegten Gemisch.

<u>Beispiele</u>

Beispiel 1

1,9-Decadien (51 g) wurden in 150 ml Toluol in einem Rührautoklaven mit 7,9 g Triphenylphosphin und 0,48 g Hydridotris-triphenylphosphin-rhodium-carbonyl vorgelegt und dann unter 3 bar Druck eine Mischung aus gleichen Volumenteilen Wasserstoff und Kohlenmonoxid eingespeist. Die Temperatur im Autoklaven wurde hierbei auf 80 °C gehalten. Nach 70 Minuten wurde kein Gas mehr aufgenommen und die Einspeisung beendet. Die NMR-Analyse erbrachte, daß 98 % des 1,9-Decadiens umgesetzt waren. Das Umsetzungsgemisch, das 1,12-Dodecandial enthielt, wurde bei einer Temperatur von 125 - 130 °C und einem Druck < 2 mbar vom Katalysator abdestilliert. Die Ausbeute an 1,12-Dodecandial betrug 57 g (82 %).

Beispiel 2

Es wurde wie im Beispiel 1 verfahren, jedoch wurde Reaktionsgemisch nach Beendigung der Gasaufnahme mit 0,87 g Cobalt-(II)-acetat versetzt, auf 60 °C erwärmt, mit 2 ml 25 %-iger Perpropionsäure versetzt und anschließend etwa 5 h mit Sauerstoff begast. Beim Abkühlen kristallisiert aus dem Reaktionsgemisch die Dodecandi säure aus. Die Ausbeute, bezogen auf eingesetztes 1,9-Decadien, betrug 76 %.

Beispiel 3

5 g 1,12-Dodecandial, wie im Beispiel synthetisiert, gelöst in 50 ml Toluol, wurden bei 60 °C mit 30 mg Cobalt-(II)-acetat und 1 ml 25 %-iger Perpropionsäure versetzt und anschließend 3 h mit Sauerstoff begast. Nach Abzug des Lösungsmittels konnte 1,12-Dodecandisäure in einer Ausbeute von 80 %, bezogen auf 1,12-Dodecandial durch Umkristallisation gewonnen werden.

## Patentansprüche

1. Verfahren zur Herstellung von 1,12-Dodecandisäure durch Hydroformylierung eines Olefins in Gegenwart eines Rhodiumkatalysators und Oxidation des dabei gebildeten Aldehyds mit Sauerstoff, dadurch gekennzeichnet, daß man 1,9-Decadien in Gegenwart von Hydridotris-triphenylphosphin-rhodium-carbonyl kombiniert mit Triphenylphosphin und/oder Triphenylphosphit hydroformyliert, und das dabei gebildete 1,12-Dodecandial in Gegenwart von anorganischen oder organischen Co(II)-salzen mit Sauerstoff oxidiert.

2. Verfahren gemäß Anspruch 1, dadurch gekennzeichnet, daß man das 1,12-Dodecandial aus dem Reaktionsgemisch abtrennt und anschließend oxidiert.

3. Verfahren gemäß Anspruch 1, dadurch gekennzeichnet, daß man das 1,12-Dodecandial im Reaktionsgemisch oxidiert.

4. Verfahren nach Anspruch 1, dadurch gekennzeichnet, daß man je Gewichtsteil des 1,9-Decadiens 0,002 bis 0,01 Gewichtsteile des Hydridotris-triphenylphosphin-rhodium-carbonyls und 0,02 bis 0,3 Gewichtsteile des Triphenylphosphins oder Triphenylphosphits anwendet.

5. Verfahren nach den Ansprüchen 1 und 4, dadurch gekennzeichnet, daß man bei Temperaturen von 70 bis 140 °C und Drücken von 1 bis 12 bar hydroformyliert.

6. Verfahren nach den Ansprüchen 1 bis 3, dadurch gekennzeichnet, daß man bei Temperaturen von 10 bis 100 °C, vorzugsweise 40 bis 90 °C, oxidiert.

7. Verfahren nach den Ansprüchen 1 bis 3, 5 und 6, dadurch gekennzeichnet, daß man je Gewichtsteil des 1,12-Dodecandials 0,005 bis 0,09 Gewichtsteile des Co(II)-salzes und 0,002 bis 0,2 Gewichtsteile einer Percarbonsäure verwendet.

8. Verfahren nach den Ansprüchen 1 bis 3, 5, 6 und 7, dadurch gekennzeichnet, daß man Co(II)-acetat verwendet.

## Claims

1. Process for the preparation of 1,12-dodecane-dioic acid by hydoformylation of an olefin in the presence of a rhodium catalyst and oxidation of the resulting aldehyde with oxygen, characterized in that 1,9-decadiene is hydroformylated in the presence of hydridotristriphenylphosphine-rhodium-carbonyl combined with triphenylphosphine and/or triphenyl phosphite and the 1,12-dodecanedial formed in this reaction is oxidized with oxygen in the presence of inorganic or organic Co-(II) salts.

2. Process according to claim 1, characterized in that 1,12-dodecanedial is separated off from the reaction mixture and then oxidized.

3. Process according to claim 1, characterized in that the 1,12-dodecanedial is oxidized in the reaction mixture.

4. Process according to claim 1, characterized in that 0.002 to 0.01 part by weight hydriodotris-triphenylphosphine-rhodium-carbonyl and 0.02 to 0.3 part by weight triphenylphosphine or triphenyl phosphite are used per part by weight of the 1,9-decadiene.

5. Process according to claims 1 and 4, characterized in that the hydroformylation is carried out at temperatures from 70 to 140°C, under pressures of 1 to 12 bar.

6. Process according to claims 1 to 3, characterized in that the oxidation is carried out at temperatures from 10 to 100°C, perferably 40 to 90°C.

7. Process according to claims 1 to 3, 5 and 6, characterized in that 0.005 to 0.09 part by weight Co(II) salt and 0.002 to 0.2 part by weight of of a percarboxylic acid are used per part by weight of the 1,12-dodecanedial.

8. Process according to claims 1 to 3, 5, 6 and 7, characterized in that Co(II) acetate is used.

## Revendications

1. Procédé pour la préparation de l'acide 1,12-décanedioïque par hydroformylation d'une oléfine en présence d'un composé à base de rhodium en tant que catalyseur et oxydation à l'aide d'oxygène de l'aldehyde ainsi formé, caractérisé en ce que du 1,9-décadiène est soumis à une hydroformylation en présence d'hydruro-tris-triphénylphosphine-rhodium-carbonyle en association avec de la triphénylphosphine et/ou du phosphite de triméthyle, et on oxyde à l'aide d'oxygène le 1,12-dodécanedial ainsi formé, en présence de sels cobalteux organiques ou minéraux.

2. Procédé selon la revendication 1, caractérisé en ce que l'on sépare le 1,12-dodécanedial avec le mélange réactionnel et ensuite on l'oxyde.

3. Procédé selon la revendication 1, caractérisé en ce que le 1,12-dodécanedial est oxydé dans le mélange réactionnel.

4. Procédé selon la revendication 1, caractérisé en ce qu'il est utilisé de 0,002 à 0,01 partie en poids de l'hydruro-tris-triphénylphosphine-rhodium-carbonyl et de 0,02 à 0,3 partie en poids du triphénylphosphine ou du phosphite de triphényle, par partie en poids du 1,9-décadiène.

5. Procédé selon l'une quelconque des revendications 1 à 4, caractérisé en ce que l'hydroformylation est effectuée à des températures de 70 à 140°C et sous des pressions de 1 à 12 bars.

6. Procédé selon l'une quelconque des revendications 1 à 3, caractérisé en ce que l'oxydation est effectuée à des températures de 10 à 100°C, de préférence de 40 à 90°C.

7. Procédé selon l'une quelconque des revendications 1 à 3, 5 et 6, caractérisé en ce qu'il est utilisé de 0,005 à 0,09 partie en poids du sel cobalteux et de 0,002 à 0,2 partie en poids d'un acide percarboxylique, par partie en poids du 1,12-dodécanedial.

8. Procédé selon l'une quelconque des revendications 1 à 3, 5, et 7, caractérisé en ce que l'on utilise l'acétate cobalteux.